# EUROPEAN PATENT APPLICATION

(11) **EP 4 621 045 A1**
(43) Date of publication of application: **24.09.2025**
(21) Application number: 23891850.2
(22) Date of filing: 23.10.2023
(51) Int. Cl.: C12N 5/071, C12N 15/10

(54) **EXOSOMES CONTAINING POLYDEOXYRIBONUCLEOTIDE, COMPOSITION INCLUDING SAME, AND PREPARATION METHOD THEREFOR**

(30) Priority: 14.11.2022 KR 20220151507
(71) Applicant: STEMON Inc., Seongnam-si, Gyeonggi-do 13201 (KR)
(72) Inventor: KIM, Soon Hag, Seongnam-si, Gyeonggi-do 13589 (KR)
(74) Representative: Mewburn Ellis LLP
(86) International application number: PCT/KR2023/016450
(87) International publication number: WO 2024/106783

(57) **Abstract**

The present invention relates to polydeoxyribonucleotide (PDRN)-containing exosomes, a composition including same, and a preparation method therefor, wherein the PDRN-containing exosomes of the present invention are derived from a fish testis tissue and include high-purity, low-molecular weight PDRN. In addition, exosomes containing a significant amount of low molecular weight PDRN can be induced through a simple process of ultrasonic treatment. Furthermore, application of ultrasound to the separated exosomes can prepare PDRN exosomes that are made to have low molecular weights, whereby the exosomes exhibit high intracellular uptake rates and thus can be advantageously used for manufacturing a tissue regeneration or anti-inflammatory composition.

## Description

### TECHNICAL FIELD

The present invention relates to exosomes containing polydeoxyribonucleotide (PDRN), a composition containing the same, and a preparation method thereof. More specifically, the present invention relates to PDRN-containing exosomes derived from fish testis tissue, a composition for tissue regeneration and an anti-inflammatory cosmetic composition containing the same, and a preparation method thereof.

### BACKGROUND ART

Polydeoxyribonucleotide (PDRN) is a genetic material, which is a high-molecular weight double-stranded polymer including a phosphate and four types of bases, which are adenine, guanine, thymine, and cytosine, and having a structure in which the four types of bases, deoxyribose, and phosphate are combined to form repeating units, and additionally, it is a single-stranded polymer including four types of bases, which are adenine (A), guanine (G), cytosine (C), and uracil (U), a sugar, and a phosphate, which form repeating units.

These genetic materials, such as DNA or RNA, may be hydrolyzed and are distributed in all living organisms, either in a form bound to proteins or in a free state. Single-stranded RNA in a free state partially forms double strands by forming a hairpin structure through hydrogen bonds between complementary parts.

Compared to other tissues, animal sperm or eggs and plant growth points have relatively high PDRN content, and these genetic materials have different efficiency and effect depending whether the molecules have a low molecular weight or a high molecular weight and depending on the extraction method. The final product, PDRN, is known to be induced to have a low molecular weight with guaranteed safety, form a complex with adenosine A2 receptor, induce angiogenesis by promoting the secretion of vascular endothelial growth factor (VEGF), and activate fibroblasts, osteoblasts, and chondrocytes.

PDRN has the viscoelasticity, which is a property of polymeric materials, and exhibits a tissue repairing effect when injected intradermally. In other words, PDRN serves as a major component of a tissue repair medical mechanism of a novel concept capable of inducing regeneration of skin tissue, rather than a simple skin filler, by proliferating and increasing the activity of fibroblasts included in skin tissue and promoting the secretion of extracellular matrices such as collagen, and it exhibits a special effect in tissue regeneration and inflammation alleviation in our body.

PDRN is known to be effective for burns and chronic wounds as it stimulates the A2 purinergic receptor, a skin regeneration signal transmitter, to promote secretion of various growth factors, generate capillaries through VEGF, improve blood circulation, exhibit anti-inflammatory effects, and prevent capillary leakage, thereby promoting skin regeneration without any special side effects. In Europe, PDRN has already been approved as a pharmaceutical product and is being used in various ways, and it is also used in Korea as a prescription drug for wound treatment and tissue regeneration.

Meanwhile, exosomes are naturally secreted nanovesicles with a diameter of 30 to 200 nm, and are known to serve as important nano-mediators that transport genetic materials from cells which they are derived from. However, exosomes are currently difficult to produce in large quantities. For example, the amount of exosomes that may be obtained from 60 million mesenchymal stem cells cultured in one liter of culture medium is 1 to 2 mg based on the contained protein content. This is an amount that may be used for therapeutic experiments on several mice, and in the case of humans, it is known that the amount of exosomes needed for the treatment of graft-versus-host disease (GVHD) is 0.05 to 0.6 mg per kg of patient body weight based on the protein content. Therefore, a technology capable of increasing the yield is required in order to use exosomes clinically and commercially.

Therefore, the present invention relates to a technology capable of producing a large amount of PDRN-containing exosomes by providing ultrasound stimulation to fish testis tissue, and is expected to be able to utilize the advantages of both PDRN and exosomes.

### DETAILED DESCRIPTION OF THE INVENTION

### TECHNICAL PROBLEM

A technical problem to be solved by the present invention is to provide an exosome containing polydeoxyribonucleotide (PDRN).

In addition, another technical problem to be solved by the present invention is to provide a preparation method of a PDRN-containing exosome.

In addition, still another technical problem to be solved by the present invention is to provide a composition for tissue regeneration and an anti-inflammatory cosmetic composition containing a PDRN-containing exosome.

The technical problems to be solved by the present invention are not limited to the above-mentioned technical problems, and other technical problems that are not mentioned may be clearly understood by a person having ordinary skill in the technical field to which the present invention pertains, from the description below.

### TECHNICAL SOLUTION

In order to achieve the above-describe objects, one embodiment of the present invention provides an exosome isolated from fish testis tissue, the exosome including fish testis tissue-derived polydeoxyribonucleotide (PDRN) therein.

In an embodiment of the present invention, the PDRN may be induced to have a low molecular weight with a length less than or equal to 1 kb.

In an embodiment of the present invention, the PDRN may be induced to have a low molecular weight with a length from 100 bp to 500 bp.

In an embodiment of the present invention, the fish may be salmon or cod

In an embodiment of the present invention, the fish may be salmon, and the exosome may contain 500 pg to 1000 pg of PDRN.

In order to achieve the above-describe objects, another embodiment of the present invention provides a preparation method of a PDRN-containing exosome, the method including: a step of pulverizing fish testis tissue; a step of ultrasonicating the tissue pulverization product; and a step of isolating a PDRN-containing exosome from the ultrasonicated tissue pulverization product.

In an embodiment of the present invention, the ultrasonication may be performed at a frequency of 10 KHz to 30 KHz and at an intensity of 15% to 40% of a maximum output intensity for 5 to 35 seconds.

In an embodiment of the present invention, the PDRN may be induced to have a low molecular weight with a length less than or equal to 1 kb.

In an embodiment of the present invention, the step of isolating the PDRN-containing exosome may include: a step of centrifugating the ultrasonicated tissue pulverization product to obtain a supernatant; a step of filtering the supernatant through a filter to obtain a filtrate; and a step of centrifugating the filtrate to concentrate.

In an embodiment of the present invention, the preparation method of the PDRN-containing exosome may further include a step of ultrasonicating the isolated PDRN-containing exosome.

In an embodiment of the present invention, the ultrasonication may be performed at a frequency of 10 KHz to 30 KHz at an intensity of 15% to 40% of a maximum output intensity and for 70 to 120 seconds.

In an embodiment of the present invention, the PDRN may be induced to have a low molecular weight with a length from 100 bp to 500 bp after the step of ultrasonicating the exosome.

In order to achieve the above-describe objects, still another embodiment of the present invention provides a composition for tissue regeneration including the exosome.

In order to achieve the above-describe objects, yet another embodiment of the present invention provides anti-inflammatory composition including the exosome.

### ADVANTAGEOUS EFFECTS

The present invention relates to a polydeoxyribonucleotide (PDRN)-containing exosome, a composition including the same, and a preparation method thereof, wherein the PDRN-containing exosome of the present invention is derived from fish testis tissue and include high-purity, low-molecular weight PDRN. In addition, an exosome containing a significant amount of low-molecular weight PDRN can be derived through a simple process of ultrasonication. Furthermore, further ultrasonication of the isolated exosome can be performed to prepare a low-molecular weight PDRN exosome that exhibits a high intracellular uptake rate and thus can be effectively used for preparing a composition for tissue regeneration or an anti-inflammatory composition.

It should be understood that the effects of the present invention are not limited to the above-described effects and include all effects that may be inferred from the features of the invention described in the detailed description or claims of the present invention.

### DESCRIPTION OF THE DRAWINGS

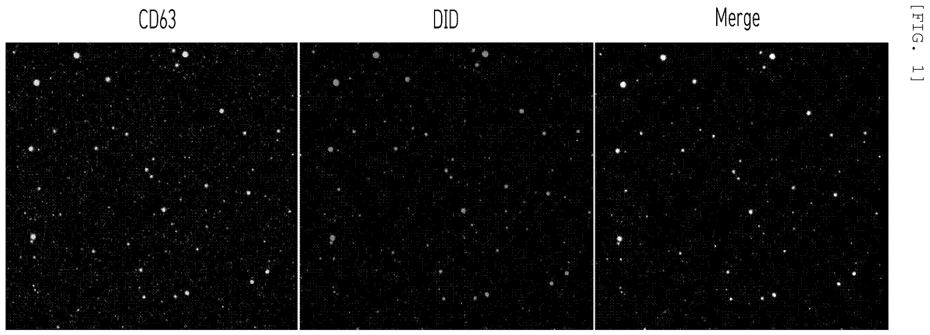

FIG. 1 shows the results of immunofluorescence imaging of salmon testis polydeoxyribonucleotide (PDRN) exosomes. The PDRN exosomes extracted from salmon testis were stained by immunofluorescence staining using phycoerythrin (PE)-labeled anti-CD63 antibody (green, H5C6, PE, eBioscience^{™}) and DID staining (red, Vybrant^{™} DID Cell-Labeling Solution; Thermo Fisher) overnight. The stained PDRN exosomes were analyzed by immunofluorescence imaging confocal microscopy (DMi8, LEICA), and the merged image was analyzed in yellow at the top.

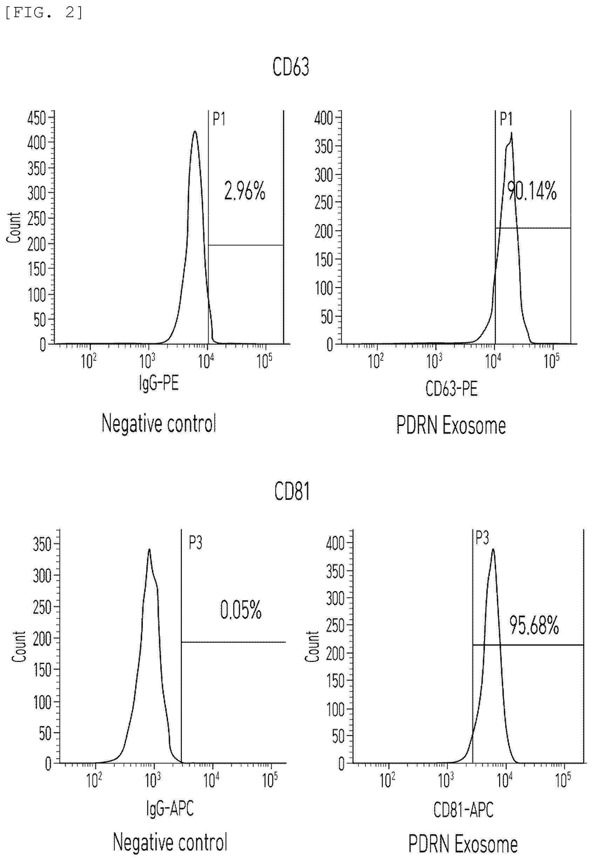

FIG. 2 shows the results of flow cytometry of the salmon testis PDRN exosomes. Ultrasonicated salmon testis PDRN exosomes were attached to aldehyde/sulfate-latex beads (A37304, Thermo Fisher) and analyzed by flow cytometry (FACSMelody^{™}, BD Biosciences) using PE-labeled CD63 (H5C6, Invitrogen) antibody and allophycocyanin (APC)-labeled CD81 (1D6, Invitrogen) antibody.

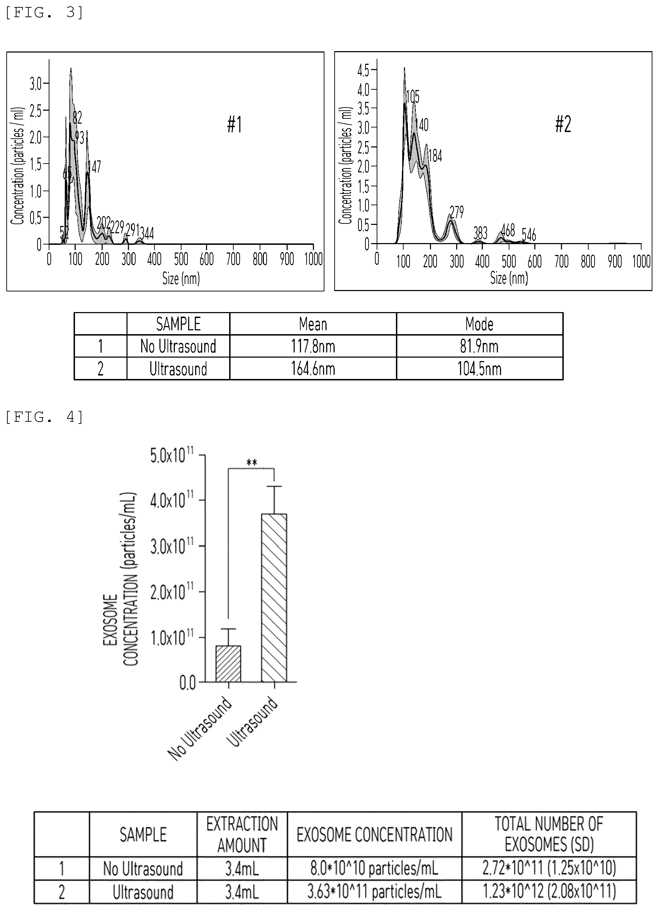

FIG. 3 shows the results of analyzing the size distribution and number of the salmon low-molecular weight PDRN exosomes. Salmon testis PDRN exosomes were isolated into two groups according to ultrasonication conditions, and the size and size distribution of the PDRN exosomes were confirmed using Nanosight. #1: Non-ultrasonicated exosomes (No Ultrasound), #2: Ultrasonicated exosomes (Ultrasound).
FIG. 4 shows the results of a comparative analysis of the number of PDRN exosomes isolated from salmon testis according to ultrasonication conditions. PDRN exosomes were isolated from salmon testis under different ultrasonication conditions, and the number of the exosomes was compared. The red bar represents the group that was not ultrasonicated (No Ultrasound), and the blue bar represents the group that was ultrasonicated (Ultrasound). * p≤0.05, ** p≤0.01.

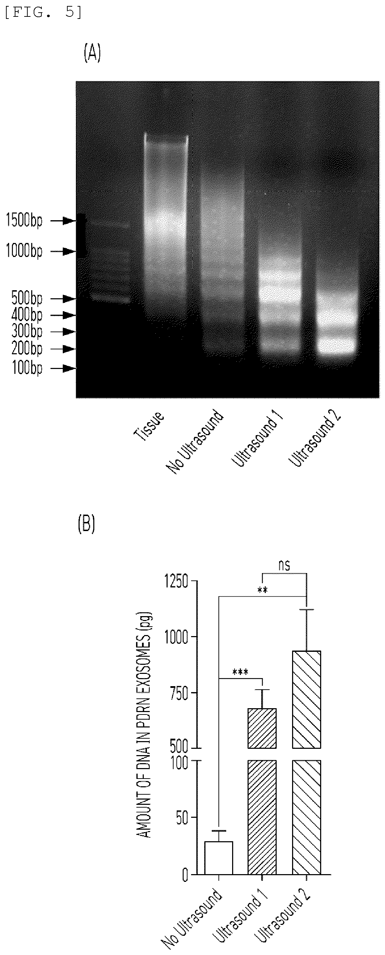

FIG. 5 shows the results of electrophoresis and content analysis of DNA included in the salmon testis low-molecular weight PDRN exosomes according to ultrasonication. (FIG. 5A) Electrophoresis was performed on a 1.3% agarose gel for 30 minutes with genomic DNA (Tissue) extracted from salmon testis (tissue lysate), DNA extracted from salmon testis PDRN exosomes that were not ultrasonicated (No Ultrasound), DNA extracted from salmon testis PDRN exosomes that were ultrasonicated once (Ultrasound 1), and DNA from salmon testis PDRN exosomes that were ultrasonicated once (Ultrasound 1) and then ultrasonicated a second time (Ultrasound 2). (FIG. 5B) The amount of DNA in exosome samples obtained by isolating PDRN exosomes from salmon testis under different ultrasonication conditions was compared. White bars represent the group that was not ultrasonicated (No ultrasound), red bars represent the group that was ultrasonicated once (Ultrasound 1), and blue bars represent the group that was ultrasonicated twice (Ultrasound 2) after exosomes were isolated from the cells. * p≤0.05, ** p≤0.01.

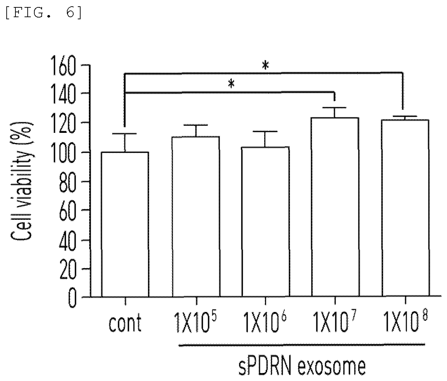

FIG. 6 shows the results of cell viability analysis of salmon low-molecular weight PDRN exosomes (sPDRN Exosomes). Human skin keratinocytes were treated with various concentrations of salmon testis PDRN exosomes (sPDRN Exosomes) induced to have a low molecular weight by ultrasonication to analyze cell viability. After culturing for 24 hours, cells were treated with Ez-cytox (DoGenBio CO., LTD., South Korea) and allowed to react for 1 hour and 30 minutes, and then the absorbance was measured at 450 nm. * p≤0.05.

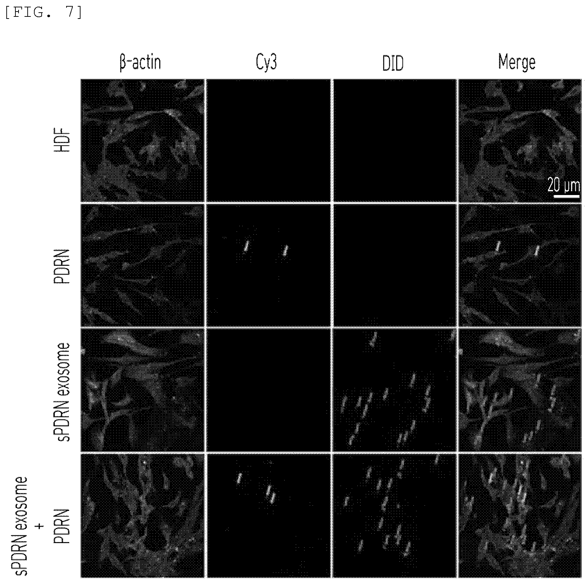

FIG. 7 shows the results of a comparative analysis of the cellular uptake of the low-molecular weight sPDRN exosomes and PDRN by an immunofluorescence analysis. To confirm the cellular uptake of the ultrasonicated low-molecular weight PDRN exosomes (Ultrasound), fluorescent-labeled PDRN and DID-stained low-molecular weight sPDRN exosomes were analyzed by immunofluorescence. The stained sPDRN exosomes were analyzed by immunofluorescence imaging confocal microscopy (DMi8, LEICA).

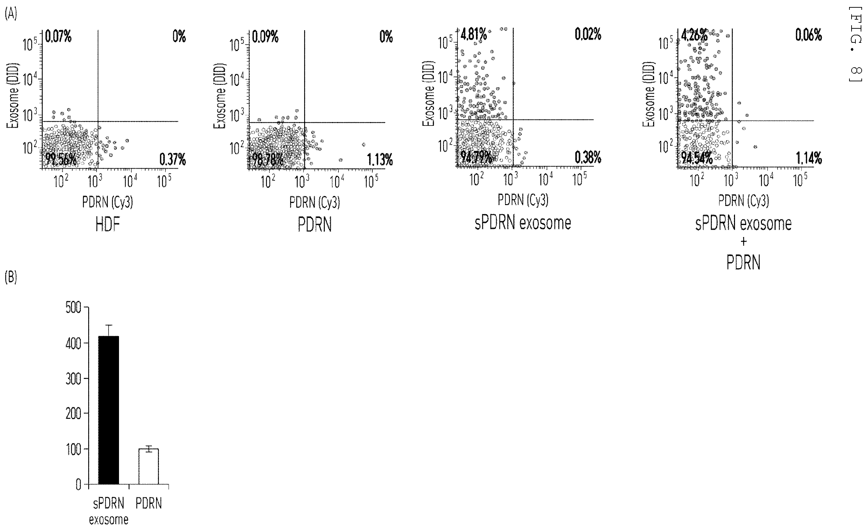

FIG. 8 shows the results of a comparative analysis of the cellular uptake of low-molecular weight sPDRN exosomes and PDRN using flow cytometry. Human fibroblasts were treated with each of 1 × 10⁹ DID-stained low-molecular weight salmon exosomes (sPDRN exosomes) and PDRN and cultured for 24 hours. Then, the measurement was performed by low cytometry (FACSMelody^{™}, BD Biosciences) on the next day. The results are presented in the flow cytometry analysis chart (FIG. 8A), and the measurement values are shown in the graph (FIG. 8B).

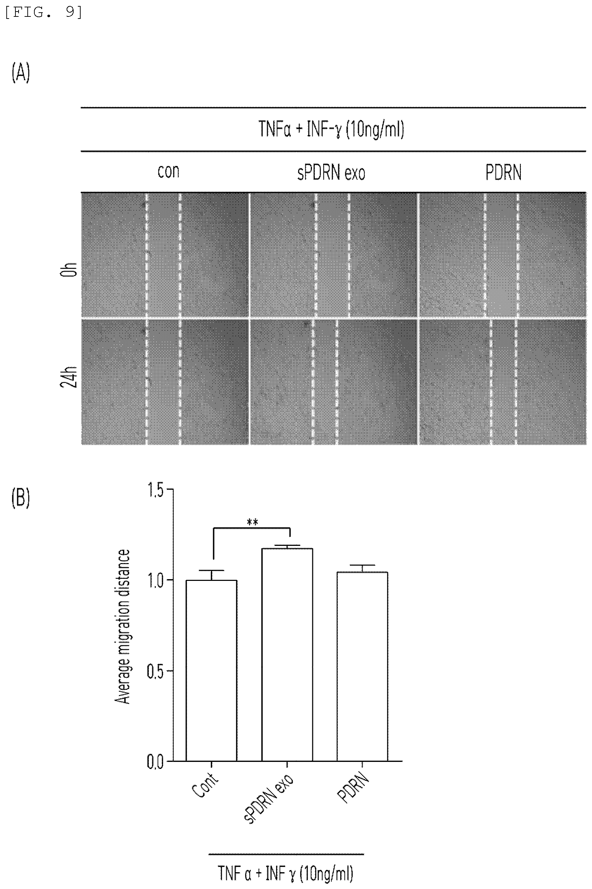

FIG. 9 shows the results of a tissue regeneration efficacy analysis of low-molecular weight sPDRN exosomes. The tissue regeneration efficacy of ultrasonicated low-molecular weight salmon PDRN exosomes was confirmed in human skin keratinocytes (HaCaT). (FIG. 9A) A migration test was performed on human skin keratinocytes. After inducing an inflammatory response with tumor necrosis factor (TNF)-α and interferon (INF)-γ for 24 hours, cells were treated with the culture medium (Cont), low-molecular weight PDRN exosomes (sPDRN exo), and PDRN, and the degree of cell migration was measured under a microscope 24 hours later. (FIG. 9B) The area of FIG. 9A was quantified using the Image J program. ** p≤0.01.

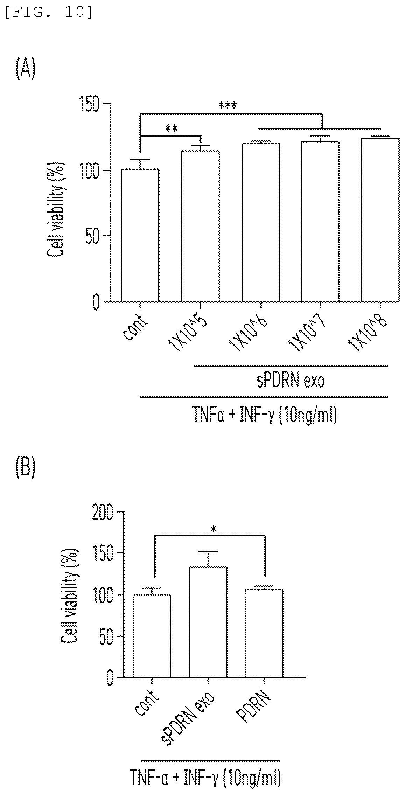

FIG. 10 shows the results of anti-inflammatory efficacy evaluation of low-molecular weight sPDRN exosomes in human skin keratinocytes. Human skin keratinocytes were simultaneously treated with TNF-α and INF-γ to induce an inflammatory response for 24 hours, and then treated with salmon testis low-molecular-weight sPDRN exosomes for 24 hours at different concentrations (FIG. 10A) and under different ultrasonication conditions (FIG. 10B) to analyze the anti-inflammatory response. * p≤0.05, ** p≤0.01, *** p≤0.001.

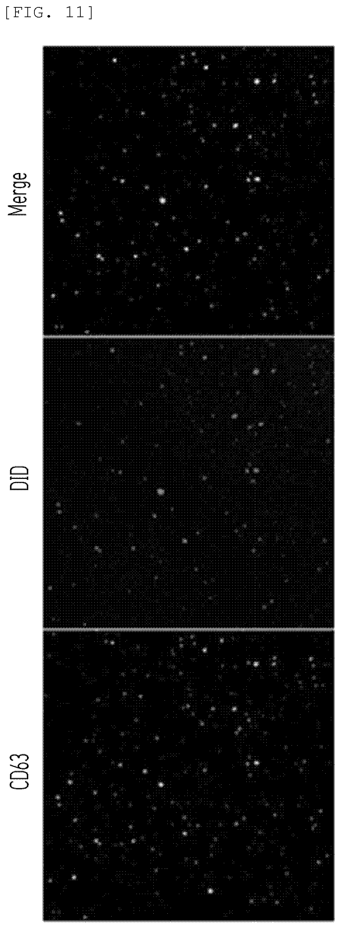

FIG. 11 shows the results of an immunofluorescence image analysis of cod testis PDRN exosomes. Immunofluorescence staining using PE-labeled anti-CD63 antibody (green, H5C6, PE, eBioscience^{™}) and DID staining (red, Vybrant^{™} DID Cell-Labeling Solution; Thermo Fisher) were performed overnight for PDRN exosomes extracted from cod testis. The stained PDRN exosomes were analyzed by immunofluorescence imaging confocal microscopy (DMi8, LEICA), and the merged image is analyzed in yellow at the top.

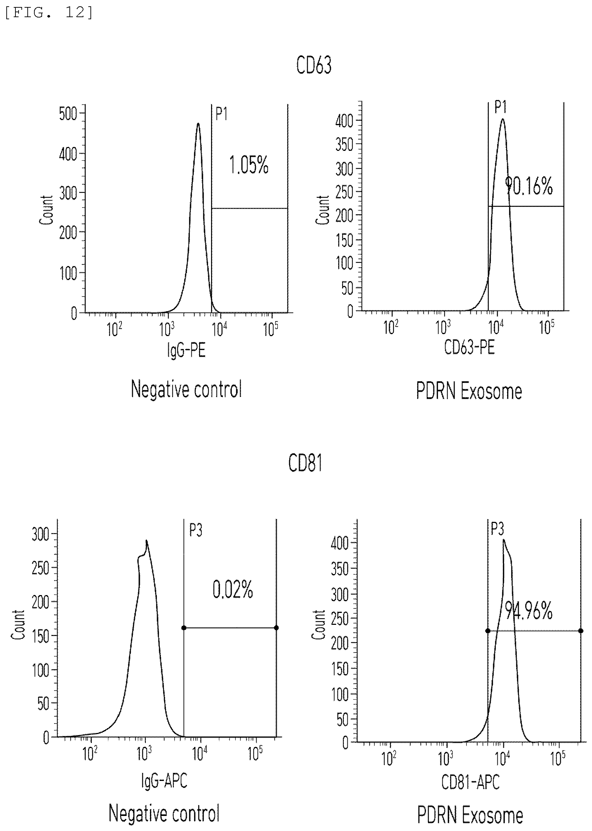

FIG. 12 shows the results of flow cytometry of the isolated cod testis PDRN exosomes. Ultrasonicated cod testis PDRN exosomes were attached to aldehyde/sulfate-latex beads (A37304, Thermo Fisher) and analyzed by flow cytometry (FACSMelody^{™}, BD Biosciences) using PE-labeled CD63 (H5C6, Invitrogen) antibody and APC-labeled CD81 (1D6, Invitrogen) antibody.

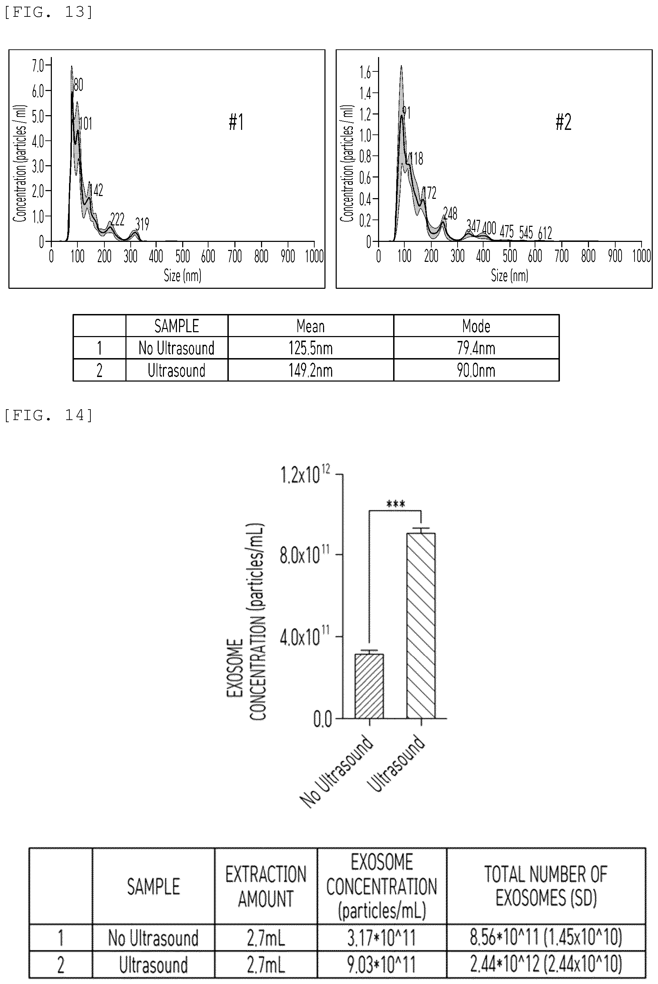

FIG. 13 shows the results of analyzing the size distribution and number of the cod low-molecular weight PDRN exosomes. Cod testis PDRN exosomes were divided into two groups according to ultrasonication conditions, and the size and size distribution of the PDRN exosomes were confirmed using Nanosight. #1: Non-ultrasonicated exosomes (No Ultrasound), #2: Ultrasonicated exosomes (Ultrasound).
FIG. 14 shows the results of a comparative analysis of the number of PDRN exosomes isolated from cod testis according to ultrasonication conditions. PDRN exosomes were isolated from cod testis under different ultrasound conditions, and the number of exosomes was compared. The red bar represents the group that was not ultrasonicated (No Ultrasound), and the blue bar represents the group that was ultrasonicated (Ultrasound). *** p≤0.001.

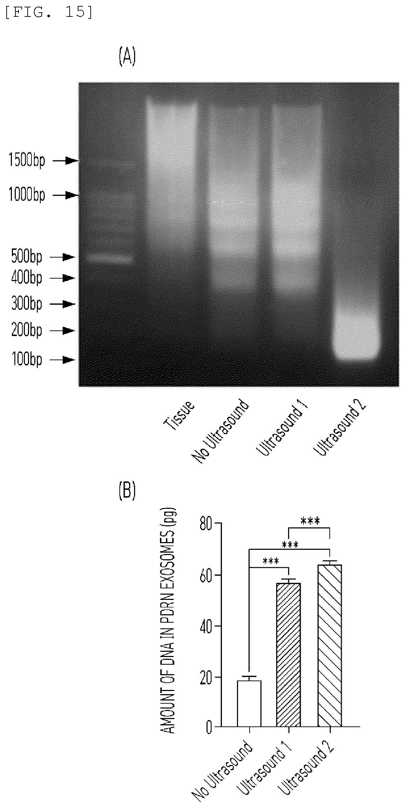

FIG. 15 shows the results of electrophoresis and content analysis DNA included in the cod testis low-molecular weight PDRN exosomes according to ultrasonication. (FIG. 15A) Electrophoresis was performed on a 1.3% agarose gel for 30 minutes with genomic DNA (Tissue) extracted from cod testis (tissue lysate), DNA extracted from cod testis PDRN exosomes that were not ultrasonicated (No Ultrasound), DNA extracted from cod testis PDRN exosomes that were ultrasonicated once (Ultrasound 1), DNA extracted from cod testis exosomes that were ultrasonicated once (Ultrasound 1) and then ultrasonicated a second time (Ultrasound 2). (FIG. 15B) The amount of DNA in exosome samples obtained by isolating PDRN exosomes from cod testis under different ultrasonication conditions was compared. Black bars represent the group that was not ultrasonicated (No ultrasound), red bars represent the group that was ultrasonicated once (Ultrasound 1), and blue bars represent the group that was ultrasonicated twice (Ultrasound 2) after exosomes were isolated from the cells. *** p≤0.001.

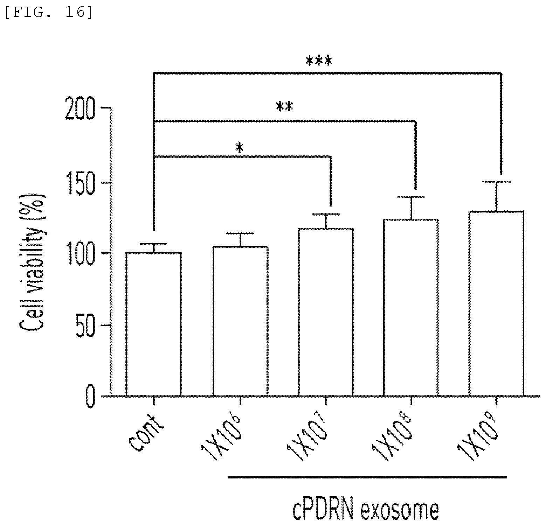

FIG. 16 shows the results of cell viability analysis of cod low-molecular PDRN exosomes (cPDRN Exosomes). Human skin keratinocytes were treated with various concentrations of cod testis PDRN exosomes (cPDRN Exosomes) induced to have a low molecular weight by ultrasonication to analyze cell viability. After culturing for 24 hours, cells were treated with Ez-cytox (DoGenBio CO., LTD., South Korea) and allowed to react for 1 hour and 30 minutes, and then the absorbance was measured at 450 nm. * p≤0.05, ** p≤0.01, ***p≤0.001.

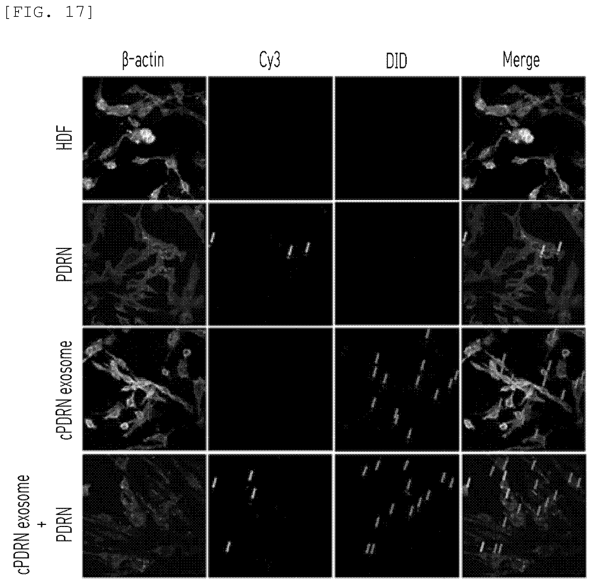

FIG. 17 shows the results of confirming the cellular uptake of the low-molecular weight cPDRN exosomes. To confirm the cellular uptake of the ultrasonicated low-molecular weight PDRN exosomes (Ultrasound), fluorescent-labeled PDRN and DID-stained low-molecular weight cPDRN exosomes were analyzed by immunofluorescence. The stained cPDRN exosomes were analyzed by immunofluorescence imaging confocal microscopy (DMi8, LEICA).

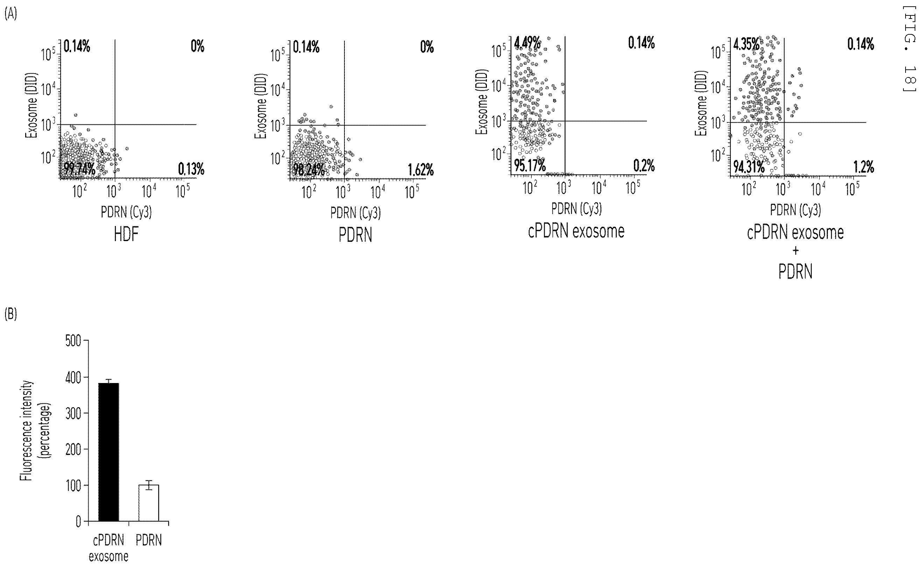

FIG. 18 shows the results of a comparative analysis of the cellular uptake of cPDRN exosomes and PDRN. Human fibroblasts were treated with each of 1 × 10⁹ DID-stained low-molecular weight cod exosomes (cPDRN exosomes) and PDRN and cultured for 24 hours. Then, the measurement was performed by low cytometry (FACSMelody^{™}, BD Biosciences) on the next day. The results are presented in the flow cytometry analysis chart (FIG. 18A), and the measurement values are shown in the graph (FIG. 18B).

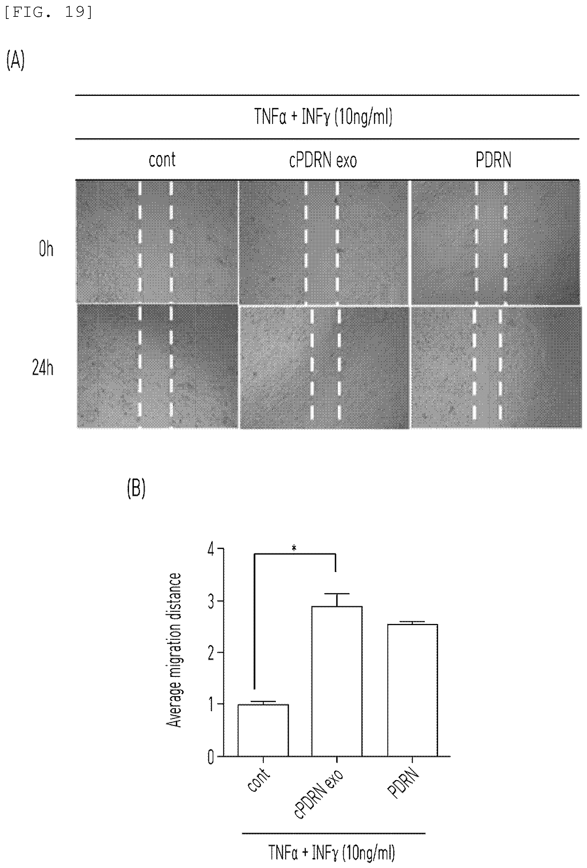

FIG. 19 shows the results of a tissue regeneration efficacy analysis of low-molecular weight cPDRN exosomes. The tissue regeneration efficacy of ultrasonicated low-molecular weight cod PDRN exosomes was confirmed in human skin keratinocytes. (FIG. 19A) A migration test was performed on human skin keratinocytes. After inducing an inflammatory response with TNF-α and INF-γ for 24 hours, the culture medium (Cont), cells were treated with the culture medium (Cont), low-molecular weight PDRN exosomes (cPDRN exo), and PDRN, and the degree of cell migration was measured under a microscope 24 hours later. (FIG. 19B) The area of FIG. 19A was quantified using the Image J program. * p≤0.05.

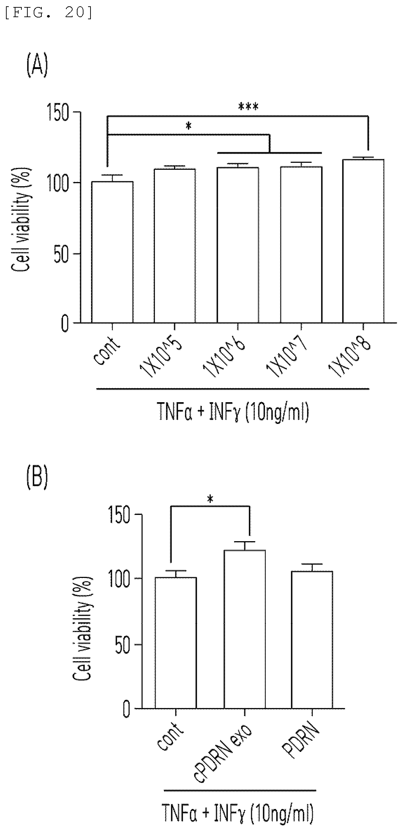

FIG. 20 shows the results of anti-inflammatory efficacy evaluation of low-molecular weight cPDRN exosomes in human skin keratinocytes. Human skin keratinocytes were simultaneously treated with TNF-α and INF-γ to induce an inflammatory response for 24 hours, and then treated with cod testis low-molecular-weight PDRN exosomes at different concentrations (FIG. 20A) and under different ultrasonication conditions (FIG. 20B) for 24 hours to analyze the anti-inflammatory response. * p≤0.05, *** p≤0.001.

### MODES OF THE INVENTION

Hereinafter, the present invention will be described in detail.

The present invention relates to an exosome isolated from fish testis tissue, the exosome including fish testis tissue-derived polydeoxyribonucleotide (PDRN) therein.

The fish refers to a vertebrate that has gills and lives in water, and may be a saltwater fish and/or a freshwater fish. For example, the fish may be a fish belonging to one or more genera of the family Salmonidae, such as genus *Oncorhynchus,* genus *Salmo,* genus *Salvelinus,* genus *Brachymystax,* and genus *Hucho,* and more specifically, it may be a salmon. In addition, the fish may be a cod belonging to the family *Gadidae,* but is not limited thereto.

The PDRN is contained in fish testis tissue, and the PDRN in the exosome of the present invention may be induced to have a low molecular weight, for example, 1 kb or less, specifically, 500 bp to 1 kb.

In addition, when an exosome isolated after ultrasonication is ultrasonicated a second time according to the method described below, the PDRN may be induced to have a low molecular weight with a length of, for example, 500 bp or less, specifically, 100 bp to 500 bp or 200 bp to 400 bp.

The exosome of the present invention contains high-purity low-molecular weight PDRN. For example, when the fish is salmon, the exosome may contain, for example, 500 pg to 1000 pg or 700 pg to 1000 pg of PDRN. In addition, when the fish is cod, the exosome may contain, for example, 50 pg to 80 pg of PDRN.

The PDRN and exosomes are derived from the tests tissue of the same fish. The PDRN is induced to have a low molecular weight by ultrasonication of the fish testis tissue, and the cells release the PDRN induced to have a low molecular weight into the extracellular environment in the form of exosomes, which may be isolated and obtained.

The present invention relates to a preparation method of a PDRN-containing exosome.

The preparation method of the present invention includes: a step of pulverizing fish testis tissue; a step of ultrasonicating the tissue pulverization product; and a step of isolating a PDRN-containing exosome from the ultrasonicated tissue pulverization product.

The fish, PDRN, and exosome are the same as described above.

The step of pulverizing fish testis tissue may be performed using a known device according to methods and conditions known in the art. For example, it may be performed by placing the fish testis in a container and grinding it, but is not limited thereto.

The ultrasonication may be performed, for example, at a frequency of 10 KHz to 30 KHz or 15 KHz to 25 KHz, at an intensity of 15% to 40% or 20% to 30% of a maximum output intensity for 5 to 35 seconds, 10 to 25 seconds, or 10 to 15 seconds. Although not limited to the above-described ranges, when the conditions are less than the above-described ranges, it is difficult to induce PDRN to have a low molecular weight, and when the conditions exceed the above-described ranges, PDRN may be induce to have an excessively low molecular weight or cells of the fish testis tissue may be completely destroyed, so that exosomes may not be isolated. Therefore, the ultrasonication may preferably be performed within the above-described ranges in order to effectively induce PDRN to have a low molecular weight and easily prepare exosomes containing high-purity PDRN.

The step of isolating the PDRN-containing exosome may be performed using a known device according to methods and conditions known in the art. For example, the step may be performed including: a step of centrifugating the ultrasonicated tissue pulverization product to obtain a supernatant; a step of filtering the supernatant through a filter to obtain a filtrate; and a step of centrifugating the filtrate to concentrate, but is not limited thereto.

The preparation method of the present invention may further include a step of ultrasonicating the isolated PDRN-containing exosome.

The ultrasonication of the exosomes may be performed, for example, at a frequency of 10 KHz to 30 KHz or 15 KHz to 25 KHz, at an intensity of 15% to 40% or 20% to 30% of a maximum output intensity, for 70 to 120 seconds, 80 to 100 seconds, or 90 to 95 seconds. By additionally ultrasonicating the isolated PDRN-containing exosome, the PDRN in the exosome may be induced to have a lower molecular weight, thereby improving the intracellular uptake.

After the step of ultrasonicating the exosome, the PDRN may be induced to have a low molecular weight with a length of, for example, 500 bp or less, specifically, 100 bp to 500 bp or 200 bp to 400 bp.

The present invention relates to a composition for tissue regeneration including the exosome.

The exosome is the same as described above.

The PDRN-containing exosome of the present invention exhibits excellent tissue regeneration ability by activating cell migration and proliferation in damaged tissue or cells compared to PDRN-containing exosomes isolated without ultrasonication.

In order to induce smoother efficacy, the composition may contain growth factors capable of stimulating smooth tissue regeneration, such as growth factors including epithelial growth factor (EGF), acid fibroblast growth factor (aFGF), basic fibroblast growth factor (bFGF), platelet derived growth factors (PDGF), transforming growth factors (TGF), leukocyte-derived growth factor (LDGF), and insulin-like growth factors (IGF), adhesion factors including fibronectin, laminin, and vitronectin, and retinoids.

Furthermore, in addition to the above-mentioned ingredients, lubricants, wetting agents, emulsifiers, suspending agents, preservatives, and the like may be further included.

Additionally, the composition may further include pharmaceutically acceptable carriers and formulations, which are described in detail in Remington's Pharmaceutical Sciences (19th ed., 1995).

The present invention relates to an anti-inflammatory cosmetic composition including the exosome.

The exosome is the same as described above.

The term "anti-inflammatory" may be used interchangeably with "inflammation suppression or amelioration" and may refer to any action that alleviates the immune response and inhibits the production of inflammatory factors.

The cosmetic composition of the present invention may be formulated as skin lotions, lotions, toners, cosmetic soaps, body wash, serums, cleansing lotions, nourishing creams, packs, massage creams, and the like, and may also be formulated as emollient toners, astringent toners, nourishing toners, eye creams, eye serums, cleansing foam, cleansing water, powder, body lotions, body creams, body oils, body serums, makeup bases, foundations, shampoos, or rinses, and the like, but is not limited thereto.

When used as a cosmetic composition, substances may be further added according to the formulation of the skin external preparation or cosmetic. For example, when the formulation is a paste, a cream, or gel, an animal oil, a vegetable oil, wax, paraffin, starch, tragacanth, cellulose derivatives, polyethylene glycol, silicone, bentonite, silica, talc or zinc oxide, or the like may be used as a carrier ingredient, and when the formulation is powder or a spray, lactose, talc, silica, aluminum hydroxide, calcium silicate or polyamide powder may be used as a carrier ingredient, and in particular, in when the formulation is a spray, it may further include a propellant such as chlorofluorohydrocarbon, propane/butane, or dimethyl ether, but it is not limited thereto. In addition, when the formulation is a solution or emulsion, a solvent, a solubilizer or an emulsifier is used as a carrier ingredient, and it may be preferably, water, ethanol, isopropanol, ethyl carbonate, ethyl acetate, benzyl alcohol, benzyl benzoate, propylene glycol, 1,3-butyl glycol oil, glycerol aliphatic esters, polyethylene glycol or fatty acid ester of sorbitan, but is not limited thereto. When the formulation is a suspension, a liquid diluent such as water, ethanol, and propylene glycol, a suspending agent such as ethoxylated isostearyl alcohol, polyoxyethylene sorbitol ester, and polyoxyethylene sorbitan ester, microcrystalline cellulose, aluminum metahydroxide, bentonite, agar, or tragacanth may be used as a carrier ingredient, but it is not limited thereto. When the formulation is a surfactant-containing cleansing agent, aliphatic alcohol sulfate, aliphatic alcohol ether sulfate, sulfosuccinic acid monoester, isethionate, imidazolinium derivatives, methyl taurate, sarcosinate, fatty acid amide ether sulfate, alkylamido betaine, aliphatic alcohols, fatty acid glyceride, fatty acid diethanolamide, vegetable oils, lanolin derivatives, or ethoxylated glycerol fatty acid ester may be used as a carrier ingredient, but it is not limited thereto.

Hereinafter, the present invention will be described in detail by way of examples to specifically explain the present invention.

### 1. Development of salmon PDRN exosomes

### (1) Isolation exosome from salmon testis

To prepare salmon PDRN exosomes with a low-molecular weight PDRN induced by ultrasonication from salmon testis, ultrasonication was performed on salmon testis tissue under the ultrasonication conditions shown in Table 1, and then exosomes were isolated and characterized.

First, 4 g of salmon testis was ground in a mortar and pestle that had been previously immersed in liquid nitrogen, 40 mL of phosphate-buffered saline (PBS) was added and mixed well. 10 mL of the resulting solution was separately taken out and stored at -20 °C (tissue lysate). The salmon testis lysate with PBS was sequentially applied using 18G, 20G, and 26G syringes, and each was moved up and down to make the volume smaller. Thereafter, 10 mL of the sample was separated and stored on ice as a non-ultrasonicated sample (No. 1, No Ultrasound). The remaining 20 mL of the salmon testis lysate sample was ultrasonicated at 25% intensity for 5 seconds three times (a total of 15 seconds).

The tissue lysate sample, the non-ultrasonicated sample (No. 1, No Ultrasound) and the sample ultrasonicated under condition 2 (No. 2, Ultrasound) was added 1.5 mL tubes in an amount of 1 mL each and centrifuged at 14,000 rpm and 4 °C for 20 minutes. The supernatants were separated and collected by group.

In order to identify the low-molecular weight PDRN exosomes derived from salmon testis according to ultrasonication in samples other than the tissue lysate sample prepared above, exosome isolation was performed as described below.

The non-ultrasonicated sample and the ultrasonicated sample were each filtered with a 0.2 µM filter. The filtered samples were placed in an Amicon^{®}Ultra-15 Centrifugal Filter Unit (UFC9100, Millipore) and centrifuged at 10,000 g, 4 °C for 30 minutes to obtain the supernatants.

**[Table 1]**

| | | Sample | Extraction method | Ultrasonication condition |
|---|---|---|---|---|
| 1 | No Ultrasound | Salmon testis tissue | Exosome isolation by tissue grinding without ultrasonication | X |
| 2 | Ultrasound | Salmon testis tissue | Exosome isolation by tissue grinding and ultrasonication | Ultrasonicated at 20 KHz, at an intensity of 25% for 15 seconds |

### (2) Identification analysis of exosomes isolated from salmon testis after ultrasonication

### (2-1) Immunofluorescence analysis of isolated salmon testis PDRN exosomes

To identify low-molecular weight PDRN exosomes isolated from salmon testis tissue after ultrasonication, an immunofluorescence analysis was performed for the exosome-specific marker 'CD63.'

For immunofluorescence staining of PDRN exosomes extracted from salmon testis, exosomes were stained using anti-CD63 antibody labeled with PE fluorescent (green, H5C6, PE, eBioscience^{™}), and lipid bilayer particle staining was performed overnight with the DID dye (red, Vybrant^{™} DID Cell-Labeling Solution; Thermo Fisher), and then the exosomes were analyzed by immunofluorescence image confocal microscopy (DMi8, LEICA) (FIG. 1).

As a result, PDRN exosomes were stained with CD63 and DID, and the two images were merged. These results showed that the isolated PDRN exosomes were typical and pure exosomes with a lipid bilayer (FIG. 1). As a result of analyzing the nanoparticles in the image using Image J, it was found that the proportion of PDRN exosomes and DID-stained particles was more than 94%, confirming that the extracted particles were high-purity exosomes.

### (2-2) Flow cytometry of isolated salmon testis PDRN exosomes

To identify low-molecular weight PDRN exosomes isolated after ultrasonication, flow cytometry (FACSMelody^{™}, BD Biosciences) was performed using exosome-specific markers CD63 (H5C6, Invitrogen) and CD81 (1D6, Invitrogen) antibodies.

0.5 µL of aldehyde/sulfate-latex beads (A37304, Thermo Fisher) were added to 50 µL of exosomes (Ultrasound) and allowed to react at room temperature for 15 minutes. After adding an additional 1 mL of 0.1% bovine serum albumin (BSA) solution, the resulting mixture was mixed well and incubated overnight at 4 °C. The next day, centrifugation was performed at 2,000 g for 10 minutes, the supernatant was removed, and the mixture was washed with 1 mL of Dulbecco's phosphate-buffered saline (DPBS). Then, centrifugation was performed again at 2,000 g for 10 minutes, and the supernatant was removed. The obtained pellet was resuspended in 50 µL DPBS.

After transferring 20 µL of the resuspended pellet to an amber tube, 80 µL of DPBS was added to make the total volume 100 µL. 5 µL of CD63 (H5C6, Invitrogen) and CD81 (1D6, Invitrogen) antibodies were added, mixed well, and allowed to react at 4 °C for one hour. After adding 1 mL of DPBS and washing, centrifugation was performed at 2,000 g for 10 minutes, the supernatant was removed, and the pellet was resuspended in 1 mL DPBS, and then measurement was performed by flow cytometry (FIG. 2).

As a result of the experiment, it was confirmed that the staining for exosome-specific markers CD63 and CD81 was more than 90%, and from the above-described results, it was confirmed that the isolated PDRN exosomes were high-purity exosome particles.

### (3) Experiment for optimization of ultrasonication conditions for isolation of salmon testis PDRN exosomes

For effective isolation of PDRN exosomes and for the isolation of an optimal low-molecular weight PDRN exosomes containing small-sized PDRN, the characteristics of the isolated PDRN exosomes were analyzed according to the presence or absence of ultrasonication and the conditions of ultrasonication.

The number and size distribution of exosomes were analyzed by nanoparticle tracking analysis (NTA) by isolating non-ultrasonicated exosomes (No Ultrasound) and ultrasonicated exosomes (Ultrasound) from salmon testis tissue, and the characteristics of PDRN contained therein were confirmed.

### (3-1) Analysis (NTA) of the number and size distribution salmon PDRN exosomes

After adjusting the final exosome volume of PDRN exosomes isolated from salmon testis to the same value, an NTA was performed using a Nanosight device (Nanosight NS300, Malvern Panalytical) to measure the size distribution of PDRN exosomes (FIG. 3).

As a result of the experiment, it was confirmed that the isolated exosomes showed a size of 50 to 200 nm, which is a typical size of exosomes, regardless of the presence or absence of ultrasonication (FIG. 3).

In addition, when the number of salmon PDRN exosomes obtained through multiple repeated tests was confirmed, the number of PDRN exosomes obtained in the ultrasonication group significantly increased by about 4.5-fold compared to the non-ultrasonication group (FIG. 4).

### (3-2) DNA analysis in salmon low-molecular weight PDRN exosomes

To confirm the amount of DNA, DNA was extracted from salmon low-molecular weight PDRN exosomes using a DNA extraction kit (iNtRON Biotechnology Inc., South Korea).

50 µL of salmon PDRN exosomes were placed in a 1.5 mL tube, 300 µL of cell lysis buffer was added and mixed well. Thereafter, 1.5 µL of RNase A solution was added, and the resulting mixture was incubated at 37 °C for 30 minutes. After adding 100 µL of protein precipitation (PPT) buffer, the resulting mixture was mixed well for 20 seconds, and centrifuged at 16,000 g and 4 °C for five minutes. 400 µL of the supernatant was collected and transferred to another 1.5 mL tube. 400 µL of isopropanol was added, mixed well, and the resulting mixture was centrifuged at 16,000 g and 4 °C for one minute. After removing the supernatant and adding 70% ethanol, the resulting mixture was well mixed, and then centrifugation was performed at 16,000 g and 4 ° C for one minute. Then, the supernatant was removed, and the pellet was dried at room temperature for about 10 minutes. Finally, 50 µL of DNA rehydration buffer was added to dissolve the DNA, and the DNA concentration was measured.

In order to confirm the DNA size distribution in the isolated PDRN exosomes, the extracted DNA was electrophoresed on an agarose gel. The DNA extracted from salmon testis tissue lysate and exosomes isolated from salmon testis without ultrasonication (No ultrasound), exosomes isolated after ultrasonication (Ultrasound 1), and exosomes isolated after ultrasonication and then ultrasonicated a second time (20 KHz, intensity 25%, 90 s) (FIG. 5A) was compared and analyzed.

1.3% agarose gel was prepared and placed in an electrophoresis device. After filling the device with a tris-acetate-EDTA (TAE) buffer to the extent that the gel was submerged, the gel was electrophoresed at 100 V for 30 minutes. Then, the gel was placed in iBright FL1000 (Invitrogen), and the DNA band was confirmed with an exposure time of 500 ms.

As a result of electrophoresis of the same amount of DNA from each group sample, the presence of PDRN in exosomes was confirmed, and it was found that the size of DNA present in exosomes was small, unlike the genomic DNA obtained from salmon testis tissue lysate (FIG. 5A, lanes 2, 3, and 4). In particular, it was found that the PDRN in the ultrasonicated exosomes was induced to have a low molecular weight, and so the size was further reduced. It was confirmed that for the exosomes isolated after performing ultrasonication once (Ultrasound 1), the DNA size was distributed around 500 bp and less than about 1.5 kb, and for the exosomes isolated after performing ultrasonication twice (Ultrasound 2), the DNA size was distributed around 200 to 400 bp bands and less than about 1 kb and (FIG. 5A).

As a result of confirming the DNA concentration in the low-molecular-weight PDRN exosomes from salmon testis, the group ultrasonicated once (Ultrasound 1) showed a significant increase by about 23.5-fold compared to the non-ultrasonicated group (No Ultrasound), and the group ultrasonicated twice (Ultrasound 2) showed a significant increase by about 32.1-fold (FIG. 5B).

In conclusion, exosomes obtained from salmon testis tissue under ultrasonication conditions were induced to have a low molecular weight compared to the non-ultrasonication group (No Ultrasound). The DNA in the low-molecular weight PDRN exosomes after ultrasonication (Ultrasound 1) was induced to have a low molecular weight and exhibited a small size of about 500 bp, and the DNA in the exosomes ultrasonicated twice (Ultrasound 2) was further induced to have a low molecular weight of about 200 to 400 bp as PDRN. Furthermore, it was confirmed that the number of exosomes increased by about 4.5-fold on average when ultrasonicated, and the PDRN content in the obtained low-molecular weight PDRN exosomes significantly increased by about 23-fold and 32-fold, respectively, depending on the ultrasonication.

Subsequent experiments (cytotoxicity evaluation, intracellular uptake analysis, and efficacy tests) were carried out using the low-molecular weight sPDRN exosomes isolated by performing ultrasonication twice (Ultrasound 2).

### (4) Analysis of cytotoxicity of PDRN exosomes derived from salmon testis

To evaluate the toxicity of PDRN exosomes (sPDRN Exosomes) from salmon testis induced to have a low molecular weight by ultrasonication, a cell viability experiment was performed. Human skin keratinocytes (HaCaT) were cultured in DMEM high-glucose culture medium supplemented with 10% fetal bovine serum and 1% penicillin/streptomycin, and 1 × 10⁴ cells were seeded per well in a 96-well plate. The next day, cells were treated with sPDRN exosomes at various concentrations (1 × 10⁴ to 1 × 10⁸ particles/mL). After 24 hours, cells were treated with 10 µL of Ez-cytox (DoGenBio CO., LTD., South Korea) was treated per 100 µL of culture medium and then allowed to react at 37 °C for 1 hour and 30 minutes in a darkened atmosphere, and the absorbance was measured at 450 nm (FIG. 6).

As a result of the experiment, when cells were treated with the low-molecular weight PDRN exosomes, there was a general tendency that the cell number increased, but no cytotoxicity was observed within the entire treatment concentration range. In particular, it was confirmed that the cell number significantly increased when the cells were treated at concentrations of 1 × 10⁷ and 1 × 10⁸ particles/mL.

### (5) Analysis of intracellular uptake of low-molecular weight PDRN exosomes and fluorescent DNA

### (5-1) Immunofluorescence analysis

To confirm the intracellular uptake of salmon exosomes induced to have a low molecular weight (sPDRN exosomes), cells were treated with Cy-3-labeled PDRN (AGC TGC TGC CTT GGC AGG AAC TAA TGG GGA TCC ATA ATA AAC CCC AGG AA-[Cyanine 3]) and DID-fluorescently stained PDRN exosomes induced to have a low molecular weight and confirmed using immunofluorescence.

After culturing human dermal fibroblasts (HDF) for three days in DMEM high-glucose culture medium supplemented with 10% fetal bovine serum and 1% penicillin/streptomycin at 37 °C, 5 × 10⁴ cells were seeded in a 24-well plate with a cover glass coated with 0.1% gelatin. PBS was added to 1 × 10⁹ exosomes to make 50 µL, 0.25 µL of DiD staining reagent was added, and the resulting mixture was mixed well and incubated at 37 °C for 30 minutes. The cells seeded in the 24-well plate were treated with 50.25 µL of DiD-stained exosomes and 0.025 µL of PDRN, and then cultured for 24 hours in an incubator kept under the conditions of 37 °C and 5% CO₂. The next day, the culture medium was aspirated and removed, and cells were washed with 1 mL of PBS, and then 1 mL of PBS was added again. Thereafter, the cover glass was taken out and moved to the humidity chamber, 200 µL of PBS was sprayed, and only 170 µL was removed so that it did not dry completely. 150 µL of 4% paraformaldehyde/PBS was sprayed on the cover glass and fixed for five minutes at room temperature. After removing 4% paraformaldehyde/PBS, 150 µL of 0.3% TritonX-100/PBS was added and permeabilization was performed. After removing 0.3% TritonX-100/PBS, 3% BSA/PBS was added, and blocking was performed for one hour at room temperature. For b-actin, the primary antibody was diluted 1:1000 in 100 µL of 3% BSA/PBS and treated for two hours at room temperature. After removing the antibody, it was washed three times with 200 µL of 0.03% Tween20/PBS. A secondary antibody was diluted 1:500 in 100 µL of 3% BSA/PBS and allowed to react at room temperature for one hour. After removing the antibody, the slide was washed three times with 200 µL of 0.03% Tween 20/PBS and twice with clean PBS. 5 µL of mounting solution was added to the slide glass, the cover glass was turned over for mounting, and after 10 minutes, the air was blocked using nail varnish, and photographs were taken under a microscope (FIG. 7).

As a result of the experiment, when the intracellular uptake of low-molecular weight sPDRN exosomes and PDRN was analyzed, it was confirmed through a confocal microscope that the exosomes containing the low-molecular weight PDRN (sPDRN exosomes) exhibited a higher cellular uptake rate than the existing PDRN.

### (5-2) Flow cytometry

To confirm the cellular uptake of salmon exosomes induced to have a low molecular weight (sPDRN exosomes) under ultrasonication conditions, cells were treated with each of Cy-3-labeled PDRN (AGC TGC TGC CTT GGC AGG AAC TAA TGG GGA TCC ATA ATA AAC CCC AGG AA- [Cyanine 3]) and DID-fluorescently stained PDRN exosomes induced to have a low molecular weight, and then confirmed by flow cytometry (FACSMelody^{™}, BD Biosciences) .

HDFs were cultured for three days in DMEM high-glucose medium supplemented with 10% fetal bovine serum and 1% penicillin/streptomycin at 37 °C, and 2 × 10⁵ cells were seeded in a 60 mm cell culture dish. To prepare dyed exosomes, PBS was added to 1 × 10⁹ exosomes to make 50 µL, 0.25 µL of DID staining reagent was added, and the resulting mixture was mixed well and incubated at 37 °C for 30 minutes. Salmon testis PDRN (100403-24-5, Sigma) was used as a positive control PDRN. The cells cultured in the 60 mm cell culture dish were treated with each or both of 50.25 µL of DID-stained salmon low-molecular weight PDRN exosomes (sPDRN Exosome) and 0.025 µL of salmon testis PDRN, and then cultured in an incubator maintained at 37 °C and 5% CO₂ for 24 h. The next day, the culture medium was aspirated and removed, and the cells were harvested by treating them with a mixture of trypsin and EDTA, centrifuged at 1000 rpm for five minutes, and the supernatant was removed. The pellet was washed with 1 mL of PBS and centrifuged at 1000 rpm for five minutes. The obtained pellet was resuspended in 1 mL of PBS and transferred to a fluorescence activated cell sorting (FACS) tube, and measurement was performed by flow cytometry (FIG. 8).

As a result of the experimental analysis of the intracellular uptake of PDRN exosomes induced to have a low molecular weight and PDRN, it was confirmed that the uptake rate was higher by approximately 4-fold in exosomes containing the low-molecular weight PDRN.

### (6) Analysis of the efficacy of low-molecular weight sPDRN exosomes

### (6-1) Analysis of the tissue regeneration efficacy of low-molecular weight PDRN exosomes

To analyze the difference in the degree to which cells proliferate into the empty space of the culture dish, a cell migration test was conducted to evaluate the regeneration efficacy of low-molecular weight sPDRN exosomes.

Human skin keratinocytes (HaCaT) were cultured in DMEM high-glucose culture medium supplemented with 10% fetal bovine serum and 1% penicillin/streptomycin at 37 °C, and 7 × 10⁴ cells were seeded per well in a 24-well plate. The next day, cells were treated with TNF-α (10 ng/ml) and INF-γ (10 ng/ml) for 24 hours in DMEM high-glucose culture medium supplemented with 2% fetal bovine serum and 1% penicillin/streptomycin. After 24 hours, the bottom of the cell culture dish was scratched with a scratcher (SPLScar^{™} Scratcher, SPL CO., LTD., South Korea) and washed twice with the medium.

Cells were treated with the cod testis low-molecular weight PDRN exosomes (sPDRN exo) isolated after ultrasonication at a concentration of 1 × 10⁷ particles/mL and with PDRN (Sigma, 31149-F) at a concentration of 70 ng/mL. Images of the scratched area were taken under a microscope at 0 h. The same area was photographed under a microscope 24 hours later (FIG. 9A), and the area was quantified using the Image J program and presented as a graph (FIG. 9B).

As a result of the experiment, it was confirmed that the tissue regeneration ability significantly increased under the ultrasonication condition (sPDRN exo) compared to the negative control group (Cont), and it was also found that the ultrasonicated exosomes exhibited an efficacy higher than or equal to that of the existing PDRN (FIG. 9).

### (6-2) Anti-inflammatory evaluation of low-molecular weight sPDRN exosomes

For the anti-inflammatory test evaluation, human skin keratinocytes (HaCaT) were used to induce inflammatory conditions, and then treated with low-molecular weight PDRN exosomes and PDRN to conduct a cell viability experiment (formazan is generated by dehydrogenase that is active only in living cells and the viability is measured by absorbance).

HaCaT cells were cultured in DMEM high-glucose culture medium supplemented with 10% fetal bovine serum and 1% penicillin/streptomycin in an incubator maintained under 37 °C and 5% CO₂ conditions. For the experiment, 1 × 10⁴ cells were seeded per well in a 96-well plate.

First, to determine the optimal concentration for anti-inflammatory efficacy of low-molecular weight PDRN exosomes, HaCaT cells were seeded, and on the next day, treated with TNF-α (10 ng/ml) and INF-γ (10 ng/ml) simultaneously and cultured in an incubator maintained at 37 °C and 5% CO₂ for 24 hours. Thereafter, the cells were treated with the low-molecular weight PDRN exosomes at various concentrations (1 × 10⁷ and 1 × 10⁸ particles/mL) for 24 hours. After treatment, the cells were treated with 10 µL of Ez-cytox (DoGenBio CO., LTD., South Korea) per 100 µL of culture medium and allowed to react at 37 °C for 1 hour and 30 minutes in a darkened atmosphere, and then the absorbance was measured at 450 nm (FIG. 10A).

Next, the anti-inflammatory efficacy of the sPDRN exosomes induced to have a low molecular weight by ultrasonication was comparatively evaluated with that of the existing PDRN at an effective concentration of 1 × 10⁷ particles/ml.

As a result, it was confirmed that the anti-inflammatory efficacy significantly increased in the groups treated with different concentrations of PDRN exosomes induced to have a low molecular weight compared to the negative control group (Cont., PBS) (FIG. 10A).

The day after seeding HaCaT cells, the cells were simultaneously treated with TNF-α (10 ng/ml) and INF-γ (10 ng/ml) and cultured for 24 hours in an incubator maintained under 37 °C and 5% CO₂ conditions. After 24 hours, the cells were treated with each of the ultrasonicated sPDRN exosome samples at a concentration of 1 × 10⁷ particles/mL and with PDRN (Sigma, 31149-F) at 70 ng/mL. After 24 hours, the cells were treated with10 µL of Ez-cytox (DoGenBio CO., LTD., South Korea) per 100 µL of culture medium and allowed to react at 37 °C for 1 hour and 30 minutes in a darkened atmosphere. The absorbance was measured at 450 nm (FIG. 10B).

As a result, it was found that the anti-inflammatory efficacy of sPDRN exosomes (sPDRN exo) induced after ultrasonication significantly increased compared to the negative control group, and an anti-inflammatory efficacy higher than or equal to that of the existing PDRN was exhibited (FIG. 10B).

### 2. Development of cod PDRN exosomes

### (1) Isolation exosome from cod testis

To prepare cod PDRN exosomes with a low-molecular weight PDRN induced by ultrasonication from cod testis, ultrasonication was performed on cod testis tissue under the ultrasonication conditions shown in Table 1, and then exosomes were isolated and characterized.

First, 4 g of cod testis was ground in a mortar and pestle that had been previously immersed in liquid nitrogen, 40 mL of phosphate-buffered saline (PBS) was added and mixed well. 10 mL of the resulting solution was separately taken out and stored at -20 °C (tissue lysate). The cod testis lysate with PBS was sequentially applied using 18G, 20G, and 26G syringes, and each was moved up and down to make the volume smaller. Thereafter, 10 mL of the sample was separated and stored on ice as a non-ultrasonicated sample (No. 1, No Ultrasound). The remaining 20 mL of the cod testis lysate sample was ultrasonicated at 25% intensity for 5 seconds three times (a total of 15 seconds).

The tissue lysate sample, the non-ultrasonicated sample (No. 1, No Ultrasound) and the sample ultrasonicated under condition 2 (No. 2, Ultrasound) was added 1.5 mL tubes in an amount of 1 mL each and centrifuged at 14,000 rpm and 4 °C for 20 minutes. The supernatants were separated and collected by group.

In order to identify the low-molecular weight PDRN exosomes derived from cod testis according to ultrasonication in samples other than the tissue lysate sample prepared above, exosome isolation was performed as described below.

The non-ultrasonicated sample and the ultrasonicated sample were each filtered with a 0.2 µM filter. The filtered samples were placed in an Amicon^{®}Ultra-15 Centrifugal Filter Unit (UFC9100, Millipore) and centrifuged at 10,000 g, 4 °C for 30 minutes to obtain the supernatants.

**[Table 2]**

| | | Sample | Extraction method | Ultrasonication condition |
|---|---|---|---|---|
| 1 | No Ultrasound | Cod testis tissue | Exosome isolation by tissue grinding without ultrasonication | X |
| 2 | Ultrasound | Cod testis tissue | Exosome isolation by tissue grinding and ultrasonication | Ultrasonicated at 20 KHz, at an intensity of 25% for 15 seconds |

### (2) Identification analysis of exosomes isolated from cod testis after ultrasonication

### (2-1) Immunofluorescence analysis of isolated cod testis PDRN exosomes

To identify low-molecular weight PDRN exosomes isolated from cod testis tissue after ultrasonication, an immunofluorescence analysis was performed for the exosome-specific marker 'CD63.'

For immunofluorescence staining of PDRN exosomes extracted from cod testis, exosomes were stained using anti-CD63 antibody labeled with PE fluorescent (green, H5C6, PE, eBioscience^{™}), and lipid bilayer particle staining was performed overnight with the DID dye (red, Vybrant^{™} DID Cell-Labeling Solution; Thermo Fisher), and then the exosomes were analyzed by immunofluorescence image confocal microscopy (DMi8, LEICA).

As a result, PDRN exosomes were stained with CD63 and DID, and the two images were merged. These results showed that the isolated PDRN exosomes were typical and pure exosomes with a lipid bilayer (FIG. 11). As a result of analyzing the nanoparticles in the image using Image J, it was found that the proportion of PDRN exosomes and DID-stained particles was more than 93%, confirming that the extracted particles were high-purity exosomes.

### (2-2) Flow cytometry of isolated cod testis PDRN exosomes

To identify low-molecular weight PDRN exosomes isolated after ultrasonication, flow cytometry (FACSMelody^{™}, BD Biosciences) was performed using exosome-specific markers CD63 (H5C6, Invitrogen) and CD81 (1D6, Invitrogen) antibodies.

0.5 µL of aldehyde/sulfate-latex beads (A37304, Thermo Fisher) were added to 50 µL of exosomes (Ultrasound) and allowed to react at room temperature for 15 minutes. After adding an additional 1 mL of 0.1% bovine serum albumin (BSA) solution, the resulting mixture was mixed well and incubated overnight at 4 °C. The next day, centrifugation was performed at 2,000 g for 10 minutes, the supernatant was removed, and the mixture was washed with 1 mL of Dulbecco's phosphate-buffered saline (DPBS). Then, centrifugation was performed again at 2,000 g for 10 minutes, and the supernatant was removed. The obtained pellet was resuspended in 50 µL DPBS.

After transferring 20 µL of the resuspended pellet to an amber tube, 80 µL of DPBS was added to make the total volume 100 µL. 5 µL of CD63 (H5C6, Invitrogen) and CD81 (1D6, Invitrogen) antibodies were added, mixed well, and allowed to react at 4 °C for one hour. After adding 1 mL of DPBS and washing, centrifugation was performed at 2,000 g for 10 minutes, the supernatant was removed, and the pellet was resuspended in 1 mL DPBS, and then measurement was performed by flow cytometry (FIG. 12).

As a result of the experiment, it was confirmed that the staining for exosome-specific markers CD63 and CD81 was more than 90%, and from the above-described results, it was confirmed that the isolated PDRN exosomes were high-purity exosome particles.

### (3) Experiment for optimization of ultrasonication conditions for isolation of cod testis PDRN exosomes

For effective isolation of PDRN exosomes and for the isolation of an optimal low-molecular weight PDRN exosomes containing small-sized PDRN, the characteristics of the isolated PDRN exosomes were analyzed according to the presence or absence of ultrasonication and the conditions of ultrasonication.

The number and size distribution of exosomes were analyzed by nanoparticle tracking analysis (NTA) by isolating non-ultrasonicated exosomes (No Ultrasound) and ultrasonicated exosomes (Ultrasound) from cod testis tissue, and the characteristics of PDRN contained therein were confirmed.

### (3-1) Analysis (NTA) of the number and size distribution cod PDRN exosomes

After adjusting the final exosome volume of PDRN exosomes isolated from cod testis to the same value, an NTA was performed using a Nanosight device (Nanosight NS300, Malvern Panalytical) to measure the size distribution and number of PDRN exosomes (FIG. 13).

As a result of the experiment, it was confirmed that the isolated exosomes showed a size of 50 to 200 nm, which is a typical size of exosomes, regardless of the presence or absence of ultrasonication (FIG. 13).

In addition, when the number of cod PDRN exosomes obtained through multiple repeated tests was confirmed, the number of PDRN exosomes obtained in the ultrasonication group significantly increased by about 2.8-fold compared to the non-ultrasonication group (FIG. 14).

### (3-2) DNA analysis in cod low-molecular weight PDRN exosomes

To confirm the amount of DNA, DNA was extracted from cod low-molecular weight PDRN exosomes using a DNA extraction kit (iNtRON Biotechnology Inc., South Korea).

50 µL of cod PDRN exosomes were placed in a 1.5 mL tube, 300 µL of cell lysis buffer was added and mixed well. Thereafter, 1.5 µL of RNase A solution was added, and the resulting mixture was incubated at 37 °C for 30 minutes. After adding 100 µL of protein precipitation (PPT) buffer, the resulting mixture was mixed well for 20 seconds, and centrifuged at 16,000 g and 4 °C for five minutes. 400 µL of the supernatant was collected and transferred to another 1.5 mL tube. 400 µL of isopropanol was added, mixed well, and the resulting mixture was centrifuged at 16,000 g and 4 °C for one minute. After removing the supernatant and adding 70% ethanol, the resulting mixture was well mixed, and then centrifugation was performed at 16,000 g and 4 ° C for one minute. Then, the supernatant was removed, and the pellet was dried at room temperature for about 10 minutes. Finally, 50 µL of DNA rehydration buffer was added to dissolve the DNA, and the DNA concentration was measured.

In order to confirm the DNA size distribution in the isolated PDRN exosomes, the extracted DNA was electrophoresed on an agarose gel. The DNA extracted from cod testis tissue lysate and exosomes isolated from cod testis without ultrasonication (No ultrasound), exosomes isolated after ultrasonication (Ultrasound 1), and exosomes isolated after ultrasonication and then ultrasonicated a second time (20 KHz, intensity 25%, 90 s) (FIG. 15A) was compared and analyzed.

1.3% agarose gel was prepared and placed in an electrophoresis device. After filling the device with a tris-acetate-EDTA (TAE) buffer to the extent that the gel was submerged, the gel was electrophoresed at 100 V for 30 minutes. Then, the gel was placed in iBright FL1000 (Invitrogen), and the DNA band was confirmed with an exposure time of 500 ms.

As a result of electrophoresis of the same amount of DNA from each group sample, the presence of PDRN in exosomes was confirmed, and it was found that the size of DNA present in exosomes was small, unlike the genomic DNA obtained from cod testis tissue lysate (FIG. 5A). In particular, it was found that the PDRN in the ultrasonicated exosomes was induced to have a low molecular weight, and so the size was further reduced. For the exosomes isolated after performing ultrasonication twice (Ultrasound 2), the DNA size was distributed around 200 to 400 bp bands and less than about 1 kb and (FIG. 15A).

As a result of confirming the DNA concentration in the low-molecular-weight PDRN exosomes from cod testis, the group ultrasonicated once (Ultrasound 1) showed a significant increase by about 3.0-fold compared to the non-ultrasonicated group (No Ultrasound), and the group ultrasonicated twice (Ultrasound 2) showed a significant increase by about 3.3-fold (FIG. 15B).

Subsequent experiments (cytotoxicity evaluation, intracellular uptake analysis, and efficacy tests) were carried out using the low-molecular weight cPDRN exosomes isolated by performing ultrasonication twice (Ultrasound 2).

### (4) Analysis of cytotoxicity of PDRN exosomes derived from cod testis

To evaluate the toxicity of PDRN exosomes (cPDRN Exosomes) from cod testis induced to have a low molecular weight by ultrasonication, a cell viability experiment was performed. Human skin keratinocytes (HaCaT) were cultured in DMEM high-glucose culture medium supplemented with 10% fetal bovine serum and 1% penicillin/streptomycin, and 1 × 10⁴ cells were seeded per well in a 96-well plate. The next day, cells were treated with cPDRN exosomes at various concentrations (1 × 10⁴ to 1 × 10⁸ particles/mL). After 24 hours, cells were treated with 10 µL of Ez-cytox (DoGenBio CO., LTD., South Korea) was treated per 100 µL of culture medium and then allowed to react at 37 °C for 1 hour and 30 minutes in a darkened atmosphere, and the absorbance was measured at 450 nm (FIG. 16).

As a result of the experiment, when cells were treated with the low-molecular weight PDRN exosomes, there was a general tendency that the cell number increased, but no cytotoxicity was observed within the entire treatment concentration range. In particular, it was confirmed that the cell number significantly increased when the cells were treated at concentrations of 1 x 10⁷ to 1 x 10⁹ particles/mL.

### (5) Analysis of intracellular uptake of low-molecular weight PDRN exosomes and fluorescent DNA

### (5-1) Immunofluorescence analysis

To confirm the intracellular uptake of cod exosomes induced to have a low molecular weight (cPDRN exosomes), cells were treated with Cy-3-labeled PDRN (AGC TGC TGC CTT GGC AGG AAC TAA TGG GGA TCC ATA ATA AAC CCC AGG AA-[Cyanine 3]) and DID-fluorescently stained PDRN exosomes induced to have a low molecular weight and confirmed using immunofluorescence.

After culturing human dermal fibroblasts (HDF) for three days in DMEM high-glucose culture medium supplemented with 10% fetal bovine serum and 1% penicillin/streptomycin at 37 °C, 5 × 10⁴ cells were seeded in a 24-well plate with a cover glass coated with 0.1% gelatin. PBS was added to 1 × 10⁹ exosomes to make 50 µL, 0.25 µL of DiD staining reagent was added, and the resulting mixture was mixed well and incubated at 37 °C for 30 minutes. The cells seeded in the 24-well plate were treated with 50.25 µL of DiD-stained exosomes and 0.025 µL of PDRN, and then cultured for 24 hours in an incubator kept under the conditions of 37 °C and 5% CO₂. The next day, the culture medium was aspirated and removed, and cells were washed with 1 mL of PBS, and then 1 mL of PBS was added again. Thereafter, the cover glass was taken out and moved to the humidity chamber, 200 µL of PBS was sprayed, and only 170 µL was removed so that it did not dry completely. 150 µL of 4% paraformaldehyde/PBS was sprayed on the cover glass and fixed for five minutes at room temperature. After removing 4% paraformaldehyde/PBS, 150 µL of 0.3% TritonX-100/PBS was added and permeabilization was performed. After removing 0.3% TritonX-100/PBS, 3% BSA/PBS was added, and blocking was performed for one hour at room temperature. For b-actin, the primary antibody was diluted 1:1000 in 100 µL of 3% BSA/PBS and treated for two hours at room temperature. After removing the antibody, it was washed three times with 200 µL of 0.03% Tween20/PBS. A secondary antibody was diluted 1:500 in 100 µL of 3% BSA/PBS and allowed to react at room temperature for one hour. After removing the antibody, the slide was washed three times with 200 µL of 0.03% Tween 20/PBS and twice with clean PBS. 5 µL of mounting solution was added to the slide glass, the cover glass was turned over for mounting, and after 10 minutes, the air was blocked using nail varnish, and photographs were taken under a microscope (FIG. 17).

As a result of the experiment, when the intracellular uptake of low-molecular weight cPDRN exosomes and PDRN was analyzed, it was confirmed through a confocal microscope that the exosomes containing the low-molecular weight PDRN (cPDRN exosomes) exhibited a higher cellular uptake rate than the existing PDRN.

### (5-2) Flow cytometry

To confirm the cellular uptake of cod exosomes induced to have a low molecular weight (cPDRN exosomes) under ultrasonication conditions, cells were treated with each of Cy-3-labeled PDRN (AGC TGC TGC CTT GGC AGG AAC TAA TGG GGA TCC ATA ATA AAC CCC AGG AA- [Cyanine 3]) and DID-fluorescently stained PDRN exosomes induced to have a low molecular weight, and then confirmed by flow cytometry (FACSMelody^{™}, BD Biosciences).

HDFs were cultured for three days in DMEM high-glucose medium supplemented with 10% fetal bovine serum and 1% penicillin/streptomycin at 37 °C, and 2 × 10⁵ cells were seeded in a 60 mm cell culture dish. To prepare dyed exosomes, PBS was added to 1 × 10⁹ exosomes to make 50 µL, 0.25 µL of DID staining reagent was added, and the resulting mixture was mixed well and incubated at 37 °C for 30 minutes. Cod testis PDRN (100403-24-5, Sigma) was used as a positive control PDRN. The cells cultured in the 60 mm cell culture dish were treated with each or both of 50.25 µL of DID-stained cod low-molecular weight PDRN exosomes (cPDRN Exosome) and 0.025 µL of cod testis PDRN, and then cultured in an incubator maintained at 37 °C and 5% CO₂ for 24 h. The next day, the culture medium was aspirated and removed, and the cells were harvested by treating them with a mixture of trypsin and EDTA, centrifuged at 1000 rpm for five minutes, and the supernatant was removed. The pellet was washed with 1 mL of PBS and centrifuged at 1000 rpm for five minutes. The obtained pellet was resuspended in 1 mL of PBS and transferred to a fluorescence activated cell sorting (FACS) tube, and measurement was performed by flow cytometry (FIG. 18).

As a result of the experimental analysis of the intracellular uptake of PDRN exosomes induced to have a low molecular weight and PDRN, it was confirmed that the uptake rate was higher by approximately 3.8-fold in exosomes containing the low-molecular weight PDRN.

### (6) Analysis of the efficacy of low-molecular weight cPDRN exosomes

### (6-1) Analysis of the tissue regeneration efficacy of low-molecular weight cPDRN exosomes

To analyze the difference in the degree to which cells proliferate into the empty space of the culture dish, a cell migration test was conducted to evaluate the regeneration efficacy of low-molecular weight cPDRN exosomes.

Human skin keratinocytes (HaCaT) were cultured in DMEM high-glucose culture medium supplemented with 10% fetal bovine serum and 1% penicillin/streptomycin at 37 °C, and 7 × 10⁴ cells were seeded per well in a 24-well plate. The next day, cells were treated with TNF-α (10 ng/ml) and INF-γ (10 ng/ml) for 24 hours in DMEM high-glucose culture medium supplemented with 2% fetal bovine serum and 1% penicillin/streptomycin. After 24 hours, the bottom of the cell culture dish was scratched with a scratcher (SPLScar^{™} Scratcher, SPL CO., LTD., South Korea) and washed twice with the medium.

Cells were treated with the cod testis low-molecular weight PDRN exosomes (cPDRN exo) isolated after ultrasonication at a concentration of 1 x 10⁷ particles/mL and with PDRN (Sigma, 31149-F) at a concentration of 70 ng/mL. Images of the scratched area were taken under a microscope at 0 h. The same area was photographed under a microscope 24 hours later (FIG. 19A), and the area was quantified using the Image J program and presented as a graph (FIG. 19B).

As a result of the experiment, it was confirmed that the tissue regeneration ability significantly increased under the ultrasonication condition (cPDRN exo) compared to the negative control group (Cont), and it was also found that the ultrasonicated exosomes exhibited an efficacy higher than or equal to that of the existing PDRN (FIG. 19).

### (6-2) Anti-inflammatory evaluation of low-molecular weight cPDRN exosomes

For the anti-inflammatory test evaluation, human skin keratinocytes (HaCaT) were used to induce inflammatory conditions, and then treated with low-molecular weight PDRN exosomes and PDRN to conduct a cell viability experiment (formazan is generated by dehydrogenase that is active only in living cells and the viability is measured by absorbance).

HaCaT cells were cultured in DMEM high-glucose culture medium supplemented with 10% fetal bovine serum and 1% penicillin/streptomycin in an incubator maintained under 37 °C and 5% CO₂ conditions. For the experiment, 1 x 10⁴ cells were seeded per well in a 96-well plate.

First, to determine the optimal concentration for anti-inflammatory efficacy of low-molecular weight PDRN exosomes, HaCaT cells were seeded, and on the next day, treated with TNF-α (10 ng/ml) and INF-γ (10 ng/ml) simultaneously and cultured in an incubator maintained at 37 °C and 5% CO₂ for 24 hours. After 24 hours, the cells were treated with the low-molecular weight PDRN exosomes at various concentrations (1 × 10⁵ to 1 × 10⁸ particles/mL) for 24 hours.

After 24 hours, the cells were treated with 10 µL of Ez-cytox (DoGenBio CO., LTD., South Korea) per 100 µL of culture medium and allowed to react at 37 °C for 1 hour and 30 minutes in a darkened atmosphere, and then the absorbance was measured at 450 nm (FIG. 20A).

As a result, it was confirmed that the anti-inflammatory efficacy significantly increased in the groups treated with PDRN exosomes induced to have a low molecular weight at different concentrations of 1 x 10⁶, 1 x 10⁷, and 1 x 10⁸ particles/mL compared to the negative control group (Cont., PBS) (FIG. 20A).

Next, the anti-inflammatory efficacy of the cPDRN exosomes induced to have a low molecular weight by ultrasonication was comparatively evaluated with that of the existing PDRN at an effective concentration of 1 x 10⁷ particles/ml.

HaCaT cells were seeded, and on the next day, the cells were treated with TNF-α (10 ng/ml) and INF-γ simultaneously and cultured in an incubator maintained at 37 °C and 5% CO₂ for 24 hours. After 24 hours, the cells were treated with the low-molecular weight cPDRN exosomes at an effective concentration (1 x 10⁷ particles/ml) for 24 hours, and treated with PDRN (Sigma, 31149-F) at 70 ng/mL. After 24 hours, the cells were treated with 10 µL of Ez-cytox (DoGenBio CO., LTD., South Korea) per 100 µL of culture medium and allowed to react at 37 °C for 1 hour and 30 minutes in a darkened atmosphere, and then the absorbance was measured at 450 nm (FIG. 20B).

As a result, it was found that the anti-inflammatory efficacy of cPDRN exosomes (cPDRN exo) induced after ultrasonication significantly increased compared to the negative control group, and an anti-inflammatory efficacy higher than or equal to that of the existing PDRN was exhibited (FIG. 20B).

## Claims

1. An exosome isolated from fish testis tissue, the exosome comprising fish testis tissue-derived polydeoxyribonucleotide (PDRN) therein.

2. The exosome according to claim 1, wherein the PDRN is induced to have a low molecular weight with a length less than or equal to 1 kb.

3. The exosome according to claim 1, wherein the PDRN is induced to have a low molecular weight with a length from 100 bp to 500 bp.

4. The exosome according to claim 1, wherein the fish is salmon or cod.

5. The exosome according to claim 1, wherein the fish is salmon, and the exosome contains 500 pg to 1000 pg of PDRN.

6. A preparation method of a polydeoxyribonucleotide (PDRN)-containing exosome, the method comprising:
a step of pulverizing fish testis tissue;
a step of ultrasonicating the tissue pulverization product; and
a step of isolating a PDRN-containing exosome from the ultrasonicated tissue pulverization product.

7. The preparation method of the PDRN-containing exosome according to claim 6, wherein the ultrasonication is performed at a frequency of 10 KHz to 30 KHz at an intensity of 15% to 40% of a maximum output intensity for 5 to 35 seconds.

8. The preparation method of the PDRN-containing exosome according to claim 6, wherein the PDRN is a low-molecular weight molecule with a length less than or equal to 1 kb.

9. The preparation method of the PDRN-containing exosome according to claim 6, wherein the step of isolating the PDRN-containing exosome includes:
a step of centrifugating the ultrasonicated tissue pulverization product to obtain a supernatant;
a step of filtering the supernatant through a filter to obtain a filtrate; and
a step of centrifugating the filtrate to concentrate.

10. The preparation method of the PDRN-containing exosome according to claim 6, further comprising a step of ultrasonicating the isolated PDRN-containing exosome.

11. The preparation method of the PDRN-containing exosome according to claim 10, wherein the ultrasonication is performed at a frequency of 10 KHz to 30 KHz at an intensity of 15% to 40% of a maximum output intensity and for 70 to 120 seconds.

12. The preparation method of the PDRN-containing exosome according to claim 10, wherein the PDRN is induced to have a low molecular weight with a length from 100 bp to 500 bp after the step of ultrasonicating the exosome.

13. A composition for tissue regeneration, comprising the exosome according to claim 1.

14. An anti-inflammatory cosmetic composition comprising the exosome according to claim 1.
